# EUROPEAN PATENT SPECIFICATION

(11) **EP 3 169 392 B1**
(45) Date of publication and mention of the grant of the patent: **03.07.2019**
(21) Application number: 15822855.1
(22) Date of filing: 16.07.2015
(51) Int. Cl.: A61M 16/06, A61M 16/00, A61M 16/08, A61M 16/22

(54) **ADJUSTABLE POSITIVE AIRWAY PRESSURE OR VENTILATION SYSTEM**
SYSTEM MIT EINSTELLBAREM ATEMWEGÜBERDRUCK ODER EINSTELLBARER BEATMUNG
SYSTÈME DE VENTILATION OU À PRESSION EXPIRATOIRE POSITIVE RÉGLABLE

(30) Priority: 16.07.2014 US 201462025073 P; 16.07.2014 US 201462025077 P; 12.09.2014 US 201462049994 P
(43) Date of publication of application: 24.05.2017
(73) Proprietor: Human Design Medical, LLC, Allston, MA 02134 (US)
(72) Inventor: HARRISON, Donald, Charlestown, Massachusetts 02129 (US); GOSLINE, Andrew, Seattle, WA 98115 (US); ARABAGI, Veaceslav, Cambridge, Massachusetts 02139 (US); KAPELUS, Aaron, Jamaica Plain, Massachusetts 02130 (US)
(74) Representative: Appleyard Lees IP LLP
(86) International application number: PCT/US2015/040724
(87) International publication number: WO 2016/011238

(56) References cited:
- US-A1- 2007 089 749
- US-A1- 2009 188 507
- US-A1- 2010 083 961
- US-A1- 2010 252 044
- US-A1- 2011 232 649
- US-A1- 2011 232 649
- US-A1- 2012 204 870
- US-A1- 2013 008 447
- US-A1- 2013 239 301
- US-B2- 8 042 539
- US-B2- 8 636 007

## Description

### BACKGROUND OF THE INVENTION

### 1. Field of the Invention

The present invention relates to medical devices, and more particularly to portions of air delivery devices that interact with the nasal passages of users. These air delivery devices may be used with positive airway pressure [PAP] such as continuous positive airway pressure [CPAP] devices, automatic positive airway pressure devices [APAP], variable positive airway pressure devices [VPAP], and bilevel positive airway pressure devices [BPAP].

### 2. Description of the Prior Art

Nasal pillows exist to be partially inserted into each of a user's nares and form a seal with the nares, which allows for the user to breathe a pressurized stream of air from the ventilator or PAP device. However, present nasal pillows have been known to have deficient seals which allow the pressurized air to escape from around the pillows and thus reduce the effectiveness of the air pressure supply. Additionally current nasal pillows often put a large and unnecessary amount of pressure on the nare region of the user's face in order to be properly held in place and form an adequate seal. Such large pressures are often required due to the limited flexibility of present nasal pillows. As such the combination of a large pressure being applied to a user's nares through an inflexible pillow can result in a large amount of discomfort which can cause insomnia, and/or greatly discourage the user's desire to use a positive air pressure device, which is often prescribed to treat potentially life threatening conditions, such as sleep apnea. As such, the continued improvement of positive air pressure facial interfaces such as masks and pillows is a continuing endeavor. US 2012/204870 is background art and discloses a breathing assistance apparatus.

A need therefore exists for a nasal pillow that is interchangeable with a mask system, flexible, adaptable to a user's nares and facial profile, and reduces pressure applied on the nare region while in use.

### SUMMARY OF THE INVENTION

The invention is defined by the claims.

A positive airway pressure assembly is provided as set out in accompanying claim 1. The positive airway pressure assembly includes a mask frame configured to support each of the nasal pillows, the mask frame receiving a supply of pressurized gas at an inlet and delivering a portion of the pressurized gas to each of the nasal pillows. The nasal pillows each include a connection interface configured to connect to the mask frame and receive the portion of pressurized gas from the mask frame therethrough. An aperture is provided on each of the nasal pillows, wherein the aperture is configured to deliver the portion of pressurized gas to one of the user's nostrils or nares.

In some embodiments each nasal pillow can be configured to taper from a narrow upper portion about the aperture to a wider base section about the connection interface forming a conical shape. In some embodiments the cone of each nasal pillow can be provided with an elliptical cross section about the narrow upper portion so as to better conform to the individual shape of the nares of a wide variety of users. The conical shape can also form an elliptical, polygonal or other shape at the base portion of each nasal pillow. In other words the nasal pillows are not limited to a circular cross-section and base portion.

In yet more embodiments the nasal pillows can be provided having an annular side wall forming a central channel through which the pressurized gas can travel. This annular side wall can have a tapering thickness being thinner at the upper portion and thicker at the base portion. Alternatively the annular side wall can be formed of a plurality of strips having a varying thickness or durometer wherein each strip extends from the aperture at the upper portion to the base portion.

An attachment sleeve is provided for interfacing between the mask frame and each of the nasal pillows. The sleeve can then provide at least one degree of motion between the mask frame and each of the pillows allowing the pillows to rotate about the mask frame by rotating the sleeve. In this embodiment each nasal pillow can be formed having a plurality of annular ribs axially spaced about a lower attachment portion of each nasal pillow, the annular ribs engaging with a corresponding recess located about attachment sleeve. These annular ribs can allow each of the nasal pillows to translate radially outward from the mask frame, i.e. axially with respect to each individual nasal pillow.

Further, and particularly for embodiments with elliptical shaped nasal pillows, each of the nasal pillows can be configured to rotate axially to adjust the angular position of each nasal pillow and achieve the most comfortable angular position for engagement with each user.

In order to interface with the attachment sleeve, each nasal pillow can be provided with an attachment portion in the form of an annular tube, the annular tube having a smaller inner diameter than the wider base section of the cone of each nasal pillow. Between the base portion of the cone and this annular tube an elastic trampoline portion can be provided which is more flexible or has a lower durometer than the cone portion and the attachment portion to allow for a certain degree of flex.

Alternatively the cone and the trampoline portions can be provided with varying thicknesses rather than durometers or materials. In such instances the cone can be provided with a wall thickness of less than 40 mils. Or in yet additional embodiments the cone can be provided with a contoured or curved outer or front surface.

In some instances the fit profile of each cone can be varied by varying the material, durometer or thickness of the cone. In one embodiment a plurality of horizontal or vertical coaxial rings are formed as part of the cone, wherein each coaxial ring has a varying durometer, thickness, or material. It will be appreciated that the portion of the cone which actually contacts the user's skin within or around the nares will often be softer to improve the comfort level for the user, as such, in the varying axial ring embodiment the durometer or thickness of each sequential coaxial ring can increases from the aperture to the wider base portion.

Also contemplated but not claimed herein is a method of providing a pressurized stream of gas to the airways of a user, the various steps including: obtaining a mask frame configured to support a plurality of nasal pillows, the mask frame receiving a supply of pressurized gas at an inlet, and delivering a portion of the pressurized gas to each of a set of apertures; selecting, from a plurality of different sized nasal pillows, a selected pair of nasal pillow best suited to fit with the nares of an intended user; affixing the selected pair of nasal pillows to the mask frame over the apertures; adjusting an individual pillow height of each pillow of the selected pair of nasal pillows with respect to the frame; adjusting a relative rotation of each pillow of the selected pair of nasal pillows; and affixing the mask frame either through, or in conjunction with the nasal pillows, to the face of the user, in a manner that the nasal pillows engage with the user's nares and provide the supply of pressurized gas to the user's airways.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIGs. 1A-B illustrate partially exploded and assembled views of a positive airway pressure assembly in accordance with various aspects of the present invention;
FIGs. 2A-B illustrate assembled and exploded views of a nasal pillow assembly for use in the positive airway pressure assembly of FIGs. 1A-B;
Figs. 3A-B illustrate cross sectional views of the nasal pillow assembly of FIGs. 2A-B which illustrate an axial translation of an individual nasal pillow;
Fig. 4 illustrates a perspective view of the nasal pillow assembly of FIGs. 2A-B which illustrate the degrees of freedom of a nasal pillow with respect to an attachment sleeve;
Fig. 5 illustrates a top view of an exemplary nasal pillow for use with the positive airway pressure assembly of FIGs. 1A-B;
FIG. 6 illustrates a bottom view of an exemplary attachment sleeve for use with the positive airway pressure assembly of FIGs. 1A-B;
FIG. 7 illustrates an alternative embodiment of an attachment sleeve having a conforming bladder for interfacing with the maxilla of the patient for use with the positive airway pressure assembly of FIGs. 1A-B;
Figs. 8A-B illustrate cross sectional views of a nasal pillow for use with the positive airway pressure assembly of FIGs. 1A-B in a resting and depressed state;
Figs. 9A-B illustrate side views of various nasal pillows having varying durometer materials, or thicknesses for achieving different user fit profiles.
FIGs. 10A-C illustrate exploded side and front views, respectively, of an alternative core or mask frame assembly for use with the ventilation and positive air pressure systems of FIGs. 1A-2B.

Reference will now be made to the exemplary embodiments illustrated, and specific language will be used herein to describe the same. It will nevertheless be understood that no limitation of the scope of the invention is thereby intended though the exemplary embodiments discussed, but the examples are for purposes of illustration of the inventive concepts.

Wherein the invention is as defined in the appended claims.

### DETAILED DESCRIPTION OF THE PREFERRED EMBODIMENT

To provide an overall understanding of the systems, devices, and methods described herein, certain illustrative embodiments will be described. Although the embodiments and features described herein are frequently described for use in connection with CPAP apparatuses, systems, and methods, it will be understood that all the components, mechanisms, systems, methods, and other features outlined below may be combined with one another in any suitable manner and may be adapted and applied to other PAP apparatuses, systems, and methods, including, but not limited to, APAP, VPAP, and BPAP apparatuses, systems, and methods.

The present application seeks to provide a solution to the aforementioned problems by creating an adjustable, comfortable, nasal pillow and mask system that is interchangeable, light-weight, and adaptable to individual users.

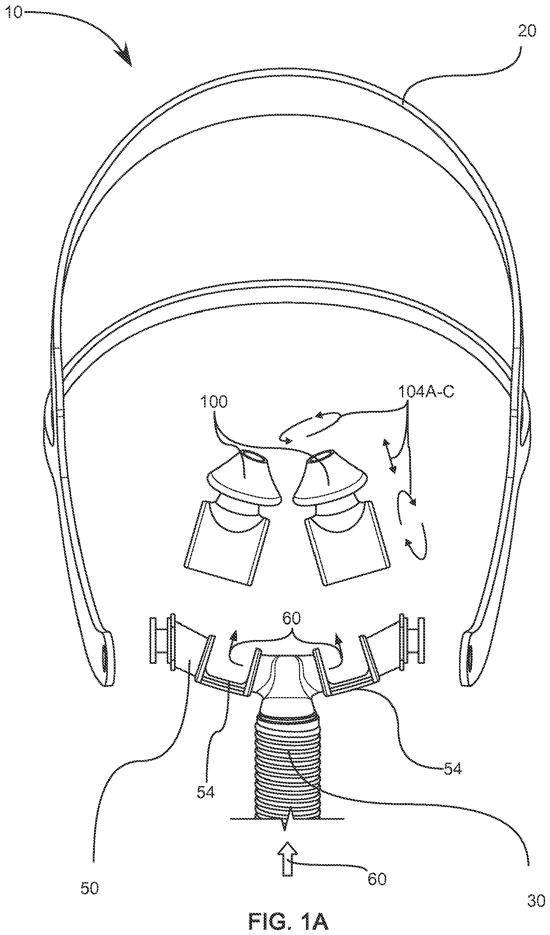

Figs. 1A-B illustrate a complete positive airway pressure assembly 10 configured to aid in supplying a stream of positive pressure air 60 to the airways of a patient wearing the assembly 10. The assembly includes a mask frame 50 having a pair of nasal pillow assemblies 100 attached thereto. The mask frame 50 receives a stream of pressurized air from a blower (not shown), which can be attached to the mask frame 50 by means of a supply hose 30. The air then travels through the mask frame 50 through apertures 54 and through the associated pillow assemblies 100 to provide air into the nostrils or nares of the user wearing the positive airway pressure assembly 10.

The positive airway pressure assembly 10 can optionally include a headgear system 20 configured to provide a sealing force between the individual pillow assemblies 100 and the nostrils of the user. In certain cases the headgear system 20 can also provide a positioning force between the mask frame 50 and the maxilla of the patient, for example on the portion of the face between the upper lip and below the nose. It will be appreciated that the headgear assembly 20 can be formed of a resilient material, or be adjustable through various means so as to conform to the individual user's contours which, understandably, vary between various users. Further, the headgear assembly 20 and can also be configured to affix to distal ends of the mask frame 50 and can be configured to provide a certain degree of rotational adjustment between the mask frame 50 and the headgear 20.

Fig. 1A also illustrates various degrees of freedom 104 achievable by the illustrated embodiment wherein each individual nasal pillow assembly 100 can rotate about a mask frame axis, the mask frame axis being defined as the axis between a central portion near the inlet and each of the distal ends. Additionally, the pillows can extend radially outward away from the mask frame 50. Finally, each individual pillow can rotate about a pillow axis being defined as an axis extending from the mask frame through a central portion of each nasal pillow assembly 100.

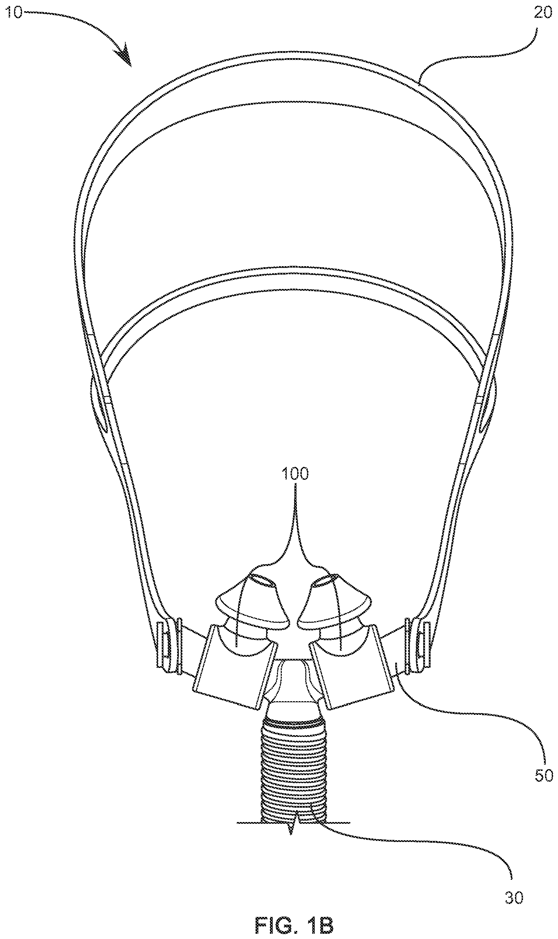

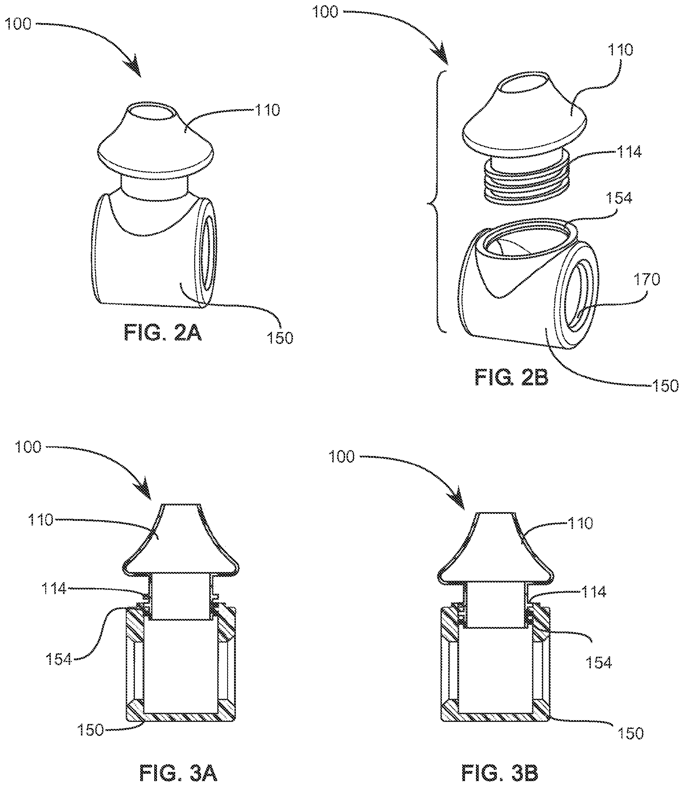

Figs. 2A-B illustrate assembled and exploded views of the nasal pillow assembly 100 which includes a nasal pillow 110 and attachment sleeves 150. The attachment sleeves 150 in this embodiment are configured to slide over the mask frame 50 and seal over apertures 54, as shown in FIG. 1, to force the air delivered to the mask frame to flow through the pillow assembly 100. The attachment sleeve 150 can be provided with an attachment portion 154 for receiving the pillow 110. The attachment portion 154 can be provided with a series of ribs or channels configured to interface with a plurality of annular ribs 114 and/or channels provided on an annular tube or stem forming an attachment portion of each pillow.

The meshing or integration of the annular ribs 114 with the channels or ribs 154 provided in the attachment sleeve allows for incremental adjustment of the relative height or radial positioning of the nasal pillow 110 with respect to the attachment sleeve 150 by changing which ribs are meshed with which respective channel. In this manner each nasal pillow can translate axially with respect to a pillow axis thus providing one degree of freedom 104A. Additionally, the ribs and channels can slide with respect to one another when twisted about the pillow axis providing a second degree of freedom 104B. Finally, the attachment sleeve 150 can be provided with a sealing lip 170 which is configured to seal against a corresponding seal provided on the mask frame 150. This sealing lip 170 allows for the attachment sleeve 150 to rotate about the mask along the mask frame axis thus providing a third degree of freedom 104C.

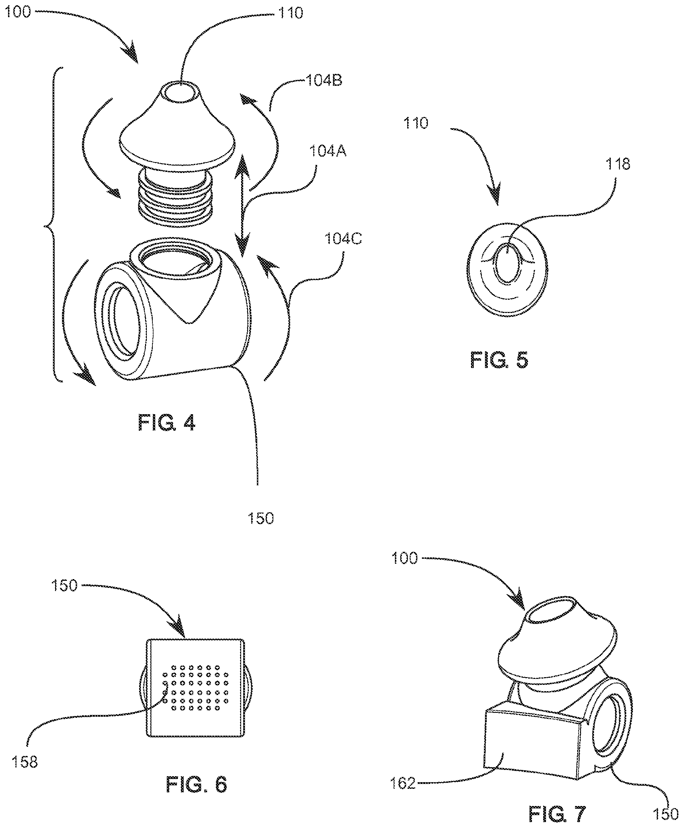

FIGs. 3A-B and FIG.4 illustrate different positions relative axial heights of the pillows 110 by incrementally meshing the ribs 114 with the channels 154 of the attachment sleeve 150, where FIG. 3A is a lower relative height and FIG. 3B is a higher relative height along the pillow axis. FIG. 4 shows all three degrees of freedom of each of the separate components as discussed.

FIG. 5 illustrates how the pillow 110 can be provided with an elliptical aperture 118 at a top or point portion which is intended to enter into the nasal passages of the wearing user. The elliptical shape, as illustrated here, is better suited to provide a seal with the nostril walls of the user. One advantage of the second degree of freedom 104B as shown in FIG. 4 is that most users actually have a mostly elliptical nostril opening, and users will have elliptical nostril openings which have varying angular positions with respect to their maxilla. By allowing the second degree of freedom 104B the relative angular position of the ellipse of the nasal pillow 110 can be adjusted so as to match the user's particular nasal openings thus providing better adjustability and customization between users.

In addition, users have differing angular locations and heights of their nasal openings from their maxilla. Thus degrees of freedom 104A and 104C allow for further customization of the relative position of the nasal pillow with respect to the nasal mask frame or attachment sleeve, either of which can be configured to rest against the user's maxilla between the nose and the upper lip.

FIG. 6 illustrates how the attachments sleeve 150 can be provided with a plurality of washout vents or apertures which allow for expiration of exhaled carbon dioxide when the user exhales. These washout vents can be provided in varying locations, including on the mask frame or at a top portion of the inlet tube, as desired.

FIG. 7 illustrates an air conform bladder 162 which can be formed as part of the attachment sleeve 150. The air conform bladder 162 can be formed of a malleable material, and have a hollow cavity defined thereby which receives pressurized gas from the interior of the attachment sleeve 150 when attached to the mask frame (not shown here). In this manner, as the pressure rises is increased when the system is on, the air conform bladder is partially inflated and acts similar to a balloon. The air conform bladder 162 can then rest against the maxilla and provide an air cushioned interface between the mask and the user's face.

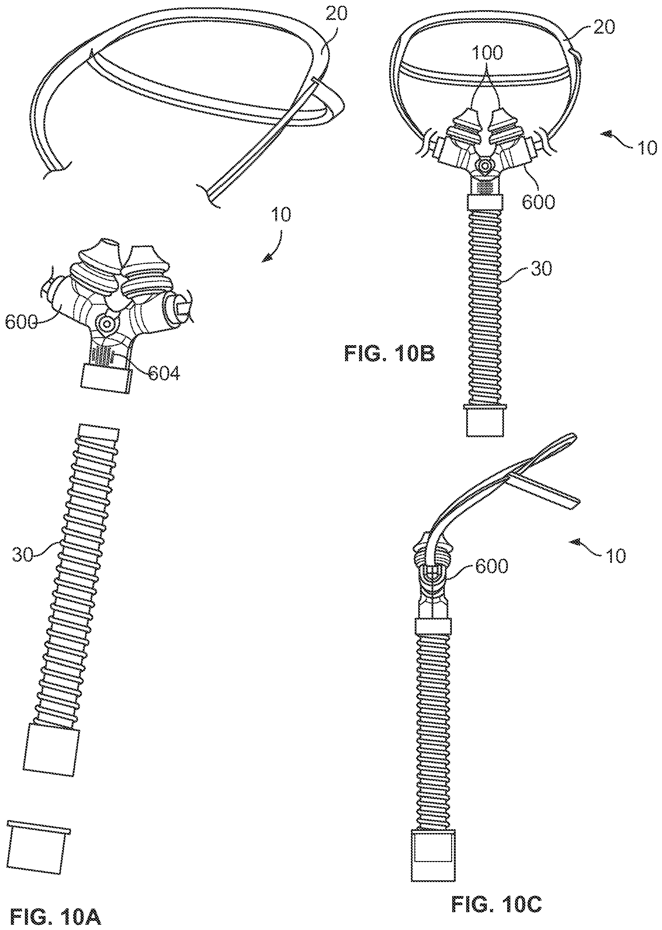

In some embodiments (not shown), such as the alternative mask frame in Figs. 10A-C, an air conform bladder can be configured to be formed as part of each nasal pillow assembly itself or as part of the mask frame.

The shape of the air conforming bladder can be curved having either a concave or convex contact surface, alternatively the contact surface can be angled, rounded or otherwise formed in any other number of desired shapes or with any number of contours so as to best engage with a user's maxilla. The malleable material, similar to the nasal pillows, can also have a varying thickness or durometer.

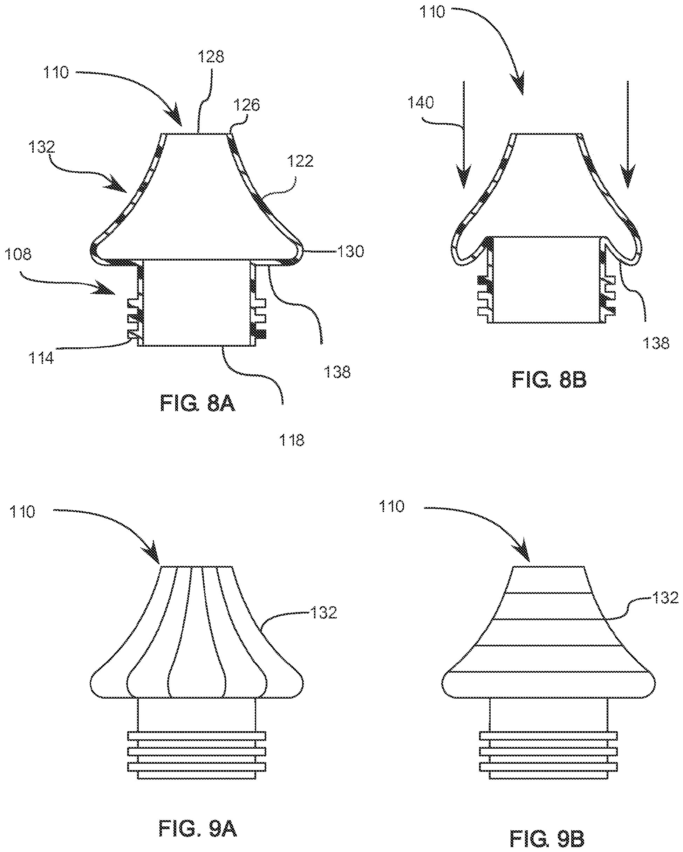

As best seen in FIGs. 8A-B, the nasal pillows 110 can be formed using an annular wall structure to provide an annular cone to interface with the users nares. The annular wall structure can have a narrower top portion 126 and a wider base portion 130 thus forming a cone structure 132 with an opening 128 for allowing air flow through a top or pinnacle of the cone structure. The outer surface or annular wall of the cone structure 132 can have varying contours so as to increase the effectiveness of the seal between the pillow's cone structure 132 and the user's nostrils. The outer surface can be curved in either a concave or a convex shape, or alternatively more complex curvatures, textures, and contours can further be provided.

The cone structure 132 can be attached to a connection interface 108 about a base portion of the nasal pillow. The base portion 108 can include an annular tube 118 with the plurality of ribs 114 as discussed above. The connection interface 108 can be attached to the cone structure using a trampoline portion 138. The trampoline portion 138 can be provided with a thinner wall or an alternative material having greater elasticity so as to allow the trampoline portion 138 to be more flexible than either the cone portion 132 or the connection interface 108. FIG. 8B illustrates how the trampoline portion 138 is allowed to flex when a sealing force is applied by the user's nostrils thus adjusting the sealing force between the nostrils and allowing the sealing force to be indirectly affected by a positioning force provided by the headgear assembly. The trampoline portion can also allow the cone portion to pivot or tilt about the annular tube or stem allowing for additional angles of adjustment. For example, Fig. 8B shows cone deforming about the trampoline portion uniformly, as a force vertically aligned with the cone is applied. However, an off vertical axial force, or alternatively a torsional force, would cause the cone to deform non-uniformly or pivot about the stem. This allows users to further customize the fitting to their individual nares.

FIGs. 9A-B illustrate how the cone portion 132 of each pillow 110 can be provided using varying thicknesses, durometers, or materials. In some embodiments the cone portion can have a thinner wall or lower durometer at a top portion and a thicker wall or a higher durometer at a base portion to provide increased comfort to the user at the portions that actually contact the inside or walls of the user's nostrils, i.e. the top portion of the cone. In some embodiments, and as shown in FIG. 9A, the cone portion 132 can be provided with a series of strips extending from a top portion to a bottom portion, each strip having varying thickness, durometer, or even varying materials so as to achieve a desired fit or comfort profile. Alternatively, as shown in FIG. 9B the cone portion 132 can be formed using a plurality of annular rings or sections, each ring or section having a different thickness, durometer, or material. In this embodiment the top ring can have a lower durometer value, or be softer than the lowest ring. The intermediate rings can gradually increase in hardness or thickness from a top portion to a base portion.

It will be appreciated that in certain embodiments the headgear can cause a direct tightening of the pillows into the nostrils of the user, thus having a direct correlation to a sealing force. In yet other embodiments, for example, when providing an air conform bladder, as discussed with reference to FIG. 7, the force applied by the headgear can be partially directed through the air conform bladder and into the maxilla to provide a primarily a positioning force, where the sealing force can be adjusted by changing the relative placement of the mask frame on the face, which is held by the positioning force. In yet additional embodiments, the nasal pillows can be caused to enter into, and hold their relative position by the elastic properties of the pillows being exerted onto the inner walls of the user's nostrils or nares without the use of headgear altogether.

The cone portion, attachment portion and the trampoline portions, as discussed above, can have varying thicknesses in the range of about 10 mils to approximately 40 mils.

In another embodiment, (not shown) the air conform bladder or cushion portion can also be filled with a foam or spongy material. This may be completely encapsulated within the sleeve or attached to the mask. In some versions the foam is open to internal air flow and pressure within the mask system. Similar to the air conforming bladder, the foam can also be shaped to fit a user's facial profile and more specifically in the area beneath the nose. It is contemplated to have detachable or interchangeable cushions of shapes and sizes to accommodate the facial features of different users.

FIGs. 10A-C illustrate an alternative embodiment of a mask frame 600. This mask frame is more rigid and instead of interfacing with the nasal pillow assembly 100 using a rotatable sleeve, the arms of mask frame 600 are rigid and do not provide rotation of the pillow assemblies 100 about the respective arm portions. This embodiment provides increased stability for headgear attachment and facial placement purposes. In this embodiment the nasal pillows are still permitted to rotate about the pillow's central axis, wherein the pillows can have an elliptical cross section. Height adjustability of each nasal pillow is also possible with some versions of mask frame 600.

The arms extending from the mask frame 600 as shown are angled and as a user rotates an elliptical-cross-sectioned nasal pillow about its axis, the angle at which the nasal pillow engages a user's nares varies. This adjustability can help a user optimize or customize the fitting to their choosing. As mentioned, the trampoline portion of the base about which the nasal pillows are formed can also deform and pivot about the stem allowing the user to customize the fit.

The rotation, non-circular cone, and pivoting features all work together to allow a customizable fit.

In this embodiment a plurality of washout vents 604 can be provided in a central portion of the mask frame 600. Additionally, the headgear 20 can be attached to the mask frame 600 using any of the previously discussed headgear attachment interfaces.

## Claims

1. A positive airway pressure assembly (10), the assembly comprising:
a plurality of nasal pillow assemblies (100);
a mask frame (50) configured to support each of the nasal pillows, the mask frame receiving a supply of pressurized gas and delivering a portion of the pressurized gas to each of the nasal pillows the mask frame including a plurality of apertures (54), each aperture being for receiving one of the nasal pillow assemblies such that the nasal pillow assembly may receive a portion of gas from said aperture;
wherein each nasal pillow assembly further comprises:
a nasal pillow (110) having a connection interface;
an attachment sleeve (150) being rotatable about the mask frame for interfacing between the mask frame and the nasal pillow, and wherein the connection interface is configured to connect to the attachment sleeve and receive the portion of pressurized gas therethrough; and
an aperture configured, in use, to deliver the portion of pressurized gas to a user's nostril;
wherein each nasal pillow tapers from a narrow upper portion about the aperture to a wider base section forming a cone, and
wherein the connection interface extends from the base portion.

2. The positive airway pressure assembly of claim 1, wherein a side wall of the cone has a constant thickness with a varying durometer between the upper portion and the base portion.

3. The positive airway pressure assembly of claim 1, wherein a side wall of the cone has a plurality of strips having a varying thickness, each strip extending from the aperture at the upper portion to the base portion.

4. The positive airway pressure assembly of claim 1, wherein each nasal pillow is provided with a plurality of annular ribs (114) axially spaced about an attachment portion of each nasal pillow, the annular ribs engaging with a corresponding recess located about the corresponding attachment sleeve.

5. The positive airway pressure assembly of claim 1, wherein each of the nasal pillows is configured to rotate axially with respect to the corresponding attachment sleeve and wherein each of the nasal pillows is also configured to incrementally translate axially with respect to the corresponding attachment sleeve.

6. The positive airway pressure assembly of claim 1, wherein the attachment sleeve can rotate axially with respect to the mask frame.

7. The positive airway pressure assembly of claim 1, wherein an attachment portion of each nasal pillow is an annular tube, the annular tube having a smaller inner diameter than the wider base section of each nasal pillow, and wherein the annular tube interfaces with the base portion of each nasal pillow with an elastic trampoline portion wherein the elastic trampoline portion has a durometer that is lower than the durometer of the annular tube and the cone.

8. The positive airway pressure assembly of claim 1, wherein an attachment portion of each nasal pillow is an annular tube, the annular tube having a smaller inner diameter than the wider base section of each nasal pillow, and wherein the annular tube interfaces with the base portion of each nasal pillow with an elastic trampoline portion wherein the elastic trampoline portion has a wall thickness lower than the wall thickness of the annular tube and the cone.

9. The positive airway pressure assembly of claim 1, wherein the cone is formed of a plurality of coaxial rings, each coaxial ring having a varying durometer.

10. The positive airway pressure assembly of claim 6, further comprising an air conforming bladder (162) extending from the attachment sleeve.

11. The positive airway pressure assembly of claim 1, wherein, for each nasal pillow assembly, the attachment sleeve is for interfacing between the mask frame, the nasal pillow and an air conforming bladder (162) extending from the attachment sleeve and configured to, in use, engage a user's maxilla.

12. The positive airway pressure assembly of claim 1, wherein the narrow upper portion has a durometer varying from at least part of the base portion.

## Patentansprüche

1. Anordnung (10) mit positivem Atemwegdruck, wobei die Anordnung Folgendes umfasst:
eine Vielzahl von Nasenkissenanordnungen (100);
einen Maskenrahmen (50), der dazu konfiguriert ist, jedes der Nasenkissen zu stützen, wobei der Maskenrahmen eine Zufuhr von mit Druck beaufschlagtem Gas aufnimmt und einen Teil des mit Druck beaufschlagten Gases an jedes der Nasenkissen liefert, wobei der Maskenrahmen eine Vielzahl von Öffnungen (54) beinhaltet, wobei jede Öffnung zum Aufnehmen einer von den Nasenkissenanordnung dient, sodass die Nasenkissenanordnung einen Teil des Gases von der Öffnung aufnehmen kann;
wobei jede Nasenkissenanordnung ferner Folgendes umfasst:
ein Nasenkissen (110), das eine Verbindungsschnittstelle aufweist;
eine Befestigungshülse (150), die um den Maskenrahmen drehbar ist, um eine Schnittstelle zwischen dem Maskenrahmen und dem Nasenkissen zu bilden, und wobei die Verbindungsschnittstelle dazu konfiguriert ist, sich mit der Befestigungshülse zu verbinden und den Teil von mit Druck beaufschlagtem Gas dadurch aufzunehmen; und
eine Öffnung, die dazu konfiguriert ist, bei Verwendung den Teil von mit Druck beaufschlagtem Gas an ein Nasenloch eines Benutzers zu liefern;
wobei sich jedes Nasenkissen von einem schmalen oberen Abschnitt um die Öffnung zu einem breiteren unteren Abschnitt verjüngt, wodurch ein Kegel gebildet wird, und
wobei sich die Verbindungsschnittstelle von dem unteren Abschnitt erstreckt.

2. Anordnung mit positivem Atemwegdruck nach Anspruch 1, wobei eine Seitenwand des Kegels eine konstante Dicke mit einem variierenden Härtegrad zwischen dem oberen Abschnitt und dem unteren Abschnitt aufweist.

3. Anordnung mit positivem Atemwegdruck nach Anspruch 1, wobei eine Seitenwand des Kegels eine Vielzahl von Streifen aufweist, die eine variierende Dicke aufweisen, wobei sich jeder Streifen von der Öffnung an dem oberen Abschnitt zu dem unteren Abschnitt erstreckt.

4. Anordnung mit positivem Atemwegdruck nach Anspruch 1, wobei jedes Nasenkissen mit einer Vielzahl von ringförmigen Rippen (114) bereitgestellt ist, die axial um einen Befestigungsabschnitt jedes Nasenkissens beabstandet sind, wobei die ringförmigen Rippen eine entsprechende Aussparung in Eingriff nehmen, die um die entsprechende Befestigungshülse gelegen ist.

5. Anordnung mit positivem Atemwegdruck nach Anspruch 1, wobei jedes der Nasenkissen dazu konfiguriert ist, sich in Bezug auf die entsprechende Befestigungshülse axial zu drehen und wobei jedes der Nasenkissen zudem dazu konfiguriert ist, sich in Bezug auf die entsprechende Befestigungshülse stufenweise axial zu verschieben.

6. Anordnung mit positivem Atemwegdruck nach Anspruch 1, wobei sich die Befestigungshülse in Bezug auf den Maskenrahmen axial drehen kann.

7. Anordnung mit positivem Atemwegdruck nach Anspruch 1, wobei ein Befestigungsteil jedes Nasenkissens ein ringförmiges Rohr ist, wobei das ringförmige Rohr einen kleineren Innendurchmesser als der breitere untere Abschnitt jedes Nasenkissens aufweist, und wobei das ringförmige Rohr über einen elastischen Trampolinabschnitt eine Schnittstelle mit dem unteren Abschnitt jedes Nasenkissens bildet, wobei der elastische Trampolinabschnitt einen Härtegrad aufweist, der geringer als der Härtegrad des ringförmigen Rohrs und des Kegels ist.

8. Anordnung mit positivem Atemwegdruck nach Anspruch 1, wobei ein Befestigungsabschnitt jedes Nasenkissens ein ringförmiges Rohr ist, wobei das ringförmige Rohr einen kleineren Innendurchmesser als der breitere untere Abschnitt jedes Nasenkissens aufweist, und wobei das ringförmige Rohr über einen elastischen Trampolinabschnitt eine Schnittstelle mit dem unteren Abschnitt jedes Nasenkissens bildet, wobei der elastische Trampolinabschnitt eine Wanddicke aufweist, die geringer als die Wanddicke des ringförmigen Rohrs und des Kegels ist.

9. Anordnung mit positivem Atemwegdruck nach Anspruch 1, wobei der Kegel aus einer Vielzahl von koaxialen Ringen gebildet ist, wobei jeder koaxiale Ring einen variierenden Härtegrad aufweist.

10. Anordnung mit positivem Atemwegdruck nach Anspruch 6, ferner umfassend einen luftanpassenden Balg (162), der sich von der Befestigungshülse erstreckt.

11. Anordnung mit positivem Atemwegdruck nach Anspruch 1, wobei bei jeder Nasenkissenanordnung die Befestigungshülse zum Bilden einer Schnittstelle zwischen der Maske, dem Nasenkissen und einem luftanpassenden Balg (162) dient, der sich von der Befestigungshülse erstreckt und dazu konfiguriert ist, bei Verwendung eine Maxilla des Benutzers in Eingriff zu nehmen.

12. Anordnung mit positivem Atemwegdruck nach Anspruch 1, wobei der schmalere obere Abschnitt einen Härtegrad aufweist, der von mindestens einem Teil des unteren Abschnitts abweicht.

## Revendications

1. Ensemble à pression expiratoire positive (10), l'ensemble comprenant :
une pluralité d'ensembles de coussins nasaux (100) ;
un cadre de masque (50) configuré pour supporter chacun des coussins nasaux, le cadre de masque recevant une alimentation en gaz sous pression et délivrant une partie du gaz sous pression à chacun des coussins nasaux, le cadre de masque comprenant une pluralité d'ouvertures (54), chaque ouverture étant destinée à recevoir l'un des ensembles de coussins nasaux de sorte que l'ensemble de coussins nasaux puisse recevoir une partie du gaz provenant de ladite ouverture ;
dans lequel chaque ensemble de coussins nasaux comprend en outre :
un coussin nasal (110) ayant une interface de raccordement ;
un manchon de fixation (150) pouvant tourner autour du cadre de masque pour servir d'interface entre le cadre de masque et le coussin nasal, et dans lequel l'interface de raccordement est configurée pour être raccordée au manchon de fixation et recevoir la partie de gaz sous pression à travers celle-ci ; et
une ouverture configurée, en cours d'utilisation, pour délivrer la partie de gaz sous pression dans la narine d'un utilisateur ;
dans lequel chaque coussin nasal est effilé d'une partie supérieure étroite autour de l'ouverture à une section de base plus large formant un cône, et
dans lequel l'interface de raccordement s'étend depuis la partie de base.

2. Ensemble à pression expiratoire positive selon la revendication 1, dans lequel une paroi latérale du cône a une épaisseur constante avec un duromètre variable entre la partie supérieure et la partie de base.

3. Ensemble à pression expiratoire positive selon la revendication 1, dans lequel une paroi latérale du cône a une pluralité de bandes ayant une épaisseur variable, chaque bande s'étendant depuis l'ouverture au niveau de la partie supérieure jusqu'à la partie de base.

4. Ensemble à pression expiratoire positive selon la revendication 1, dans lequel chaque coussin nasal est muni d'une pluralité de nervures annulaires (114) espacées axialement autour d'une partie de fixation de chaque coussin nasal, les nervures annulaires venant en prise avec un évidement correspondant situé autour du manchon de fixation correspondant.

5. Ensemble à pression expiratoire positive selon la revendication 1, dans lequel chacun des coussins nasaux est configuré pour tourner axialement par rapport au manchon de fixation correspondant et dans lequel chacun des coussins nasaux est également configuré pour effectuer une translation incrémentielle axiale par rapport au manchon de fixation correspondant.

6. Ensemble à pression expiratoire positive selon la revendication 1, dans lequel le manchon de fixation peut tourner axialement par rapport au cadre de masque.

7. Ensemble à pression expiratoire positive selon la revendication 1, dans lequel une partie de fixation de chaque coussin nasal est un tube annulaire, le tube annulaire ayant un diamètre interne inférieur à la section de base plus large de chaque coussin nasal, et dans lequel le tube annulaire sert d'interface avec la partie de base de chaque coussin nasal avec une partie de trampoline élastique, dans lequel la partie de trampoline élastique a un duromètre qui est inférieur au duromètre du tube annulaire et du cône.

8. Ensemble à pression expiratoire positive selon la revendication 1, dans lequel une partie de fixation de chaque coussin nasal est un tube annulaire, le tube annulaire ayant un diamètre interne inférieur à la section de base plus large de chaque coussin nasal, et dans lequel le tube annulaire sert d'interface avec la partie de base de chaque coussin nasal avec une partie de trampoline élastique, dans lequel la partie de trampoline élastique a une épaisseur de paroi inférieure à l'épaisseur de paroi du tube annulaire et du cône.

9. Ensemble à pression expiratoire positive selon la revendication 1, dans lequel le cône est formé d'une pluralité de bagues coaxiales, chaque bague coaxiale ayant un duromètre variable.

10. Ensemble à pression expiratoire positive selon la revendication 6, comprenant en outre un diaphragme pneumatique (162) s'étendant depuis le manchon de fixation.

11. Ensemble à pression expiratoire positive selon la revendication 1, dans lequel, pour chaque ensemble de coussins nasaux, le manchon de fixation est destiné à servir d'interface entre le cadre de masque, le coussin nasal et un diaphragme pneumatique (162) s'étendant depuis le manchon de fixation et configurée pour venir en prise avec la mâchoire d'un utilisateur.

12. Ensemble à pression expiratoire positive selon la revendication 1, dans lequel la partie supérieure étroite a un duromètre variable depuis au moins une partie de la partie de base.
